# EUROPEAN PATENT SPECIFICATION

(11) **EP 1 827 204 B1**
(45) Date of publication and mention of the grant of the patent: **05.10.2011**
(21) Application number: 05789185.5
(22) Date of filing: 19.08.2005
(51) Int. Cl.: A61B 5/00, G01N 21/64, G01J 3/28

(54) **AN INSTRUMENT WITH A MEMORY CARD UPDATING MEASURMENT ALGORITHMS AND METHODS OF USING THE SAME**
INSTRUMENT MIT SPEICHERKARTEN-AKTUALISIERUNGSMESSLALGORITHMEN UND ANWENDUNGSVERFAHREN DAFÜR
INSTRUMENT AVEC UNE CARTE MEMOIRE METTANT A JOUR DES ALGORITHMES DE MESURE ET PROCEDES D'UTILISATION DE CELUI-CI

(30) Priority: 24.08.2004 US 603951 P
(43) Date of publication of application: 05.09.2007
(60) Divisional application: 10181936.5
(73) Proprietor: Bayer HealthCare LLC, Tarrytown, NY 10591 (US)
(72) Inventor: DODSON, Neil, A., Elkhart, Illinois 46514 (US)
(74) Representative: Linhart, Angela
(86) International application number: PCT/US2005/029543
(87) International publication number: WO 2006/023721

(56) References cited:
- EP-A2- 0 640 978
- US-A- 5 077 476
- US-A- 5 899 855
- US-B1- 6 438 396

## Description

### FIELD OF THE INVENTION

The present invention generally relates to a programming strip to be used in an instrument and, more particularly, to a programming strip that is adapted to provide updated information to a processor of the instrument that determines the concentration of an analyte (e.g., glucose).

### BACKGROUND OF THE INVENTION

The quantitative determination of analytes in body fluids is of great importance in the diagnoses and maintenance of certain physiological abnormalities. For example, lactate, cholesterol and bilirubin should be monitored in certain individuals. In particular, determining glucose in body fluids is important to diabetic individuals who must frequently check the glucose level in their body fluids to regulate the glucose intake in their diets. To determine what, if any, medication (e.g., insulin) needs to be administered, a reagent-test sensor may be used for testing a fluid such as a sample of blood. The test sensors typically have been used in an instrument, including sensor-dispensing instruments. The instruments typically include a processor that is initially programmed at a manufacturing facility.

To properly read and determine the information obtained from the reagent-test sensor, new information may periodically be needed to update the processor of the instrument. This information may be in the form of reprogramming the existing algorithms, altering constraints, or installing new algorithms. One expensive option is for the user to discard the existing instrument and replace it with a new instrument that includes the updated information. Another option, which is time-consuming and expensive, is to return the existing instrument to a manufacturing site that updates the new information to the processor. The manufacturing sites generally use expensive and complicated equipment including computers.

US 5,077,476 discloses an instrument for non-invasive measurement of blood glucose. A cartridge stores data unique to an individual patient/user. The cartridge is replaceable and is plugged into the analysis instrument through a receiving port. The cartridge includes electronic memory storage devices for storing data regarding an individual user and instrument software. The memory device is adapted to store a series of glucose readings, the time and the date of all measurements made by the analysis instrument, the total number of measurements made using a particular battery, as well as calibration constants for use by data processor. The memory device may be an EPROM-type chip that contains the software for operating the analytical instrument, thus facilitating re-programming of instrument by inserting an updated cartridge. In certain embodiments the cartridge also comprises a battery for supplying power to the near-infrared quantitative analysis instrument. The memory device and the battery are in electrical communication with the processing means via interface.

It would be desirable to overcome the above-noted problems, while still being able to provide updated information to the instrument with little or no interaction by the user.

### SUMMARY OF THE INVENTION

According to one embodiment, a non-volatile memory is adapted to provide updated information to an instrument. The instrument has a processor and is adapted to determine the concentration of an analyte.
The non-volatile memory is adapted to store the updated information and is adapted to be communicatively coupled to the processor of the instrument. The non-volatile memory is removable to communicatively decouple the non-volatile memory from the processor. The instrument remains operable to determine the concentration of the analyte after the non-volatile memory is removed.

The non-volatile memory can be an EEPROM. Preferably the communication bus has exactly two lines that are adapted to assist in communicatively coupling the memory and the processor of the instrument. The first line is a power-data communication line and the second line is a ground line.

The non-volatile memory can be a flash memory.

According to one embodiment, a cartridge is adapted to be used in a sensor-dispensing instrument. The instrument is adapted to determine the concentration of an analyte and includes a processor. The cartridge comprises a programming strip and a plurality of test sensors. The programming strip includes a non-volatile memory. The non-volatile memory is adapted to store updated information and is adapted to be communicatively coupled with the processor of the instrument.
Preferably the programming strip includes a communication bus having exactly two lines that are adapted to assist in communicatively coupling the memory and the processor of the instrument. The first line is a power-data communication line and the second line is a ground line.

According to one embodiment, an instrument for determining the analyte concentration of a fluid comprises a non-volatile memory, an opening adapted to couple the non-volatile memory to the instrument and a processor. The non-volatile memory is adapted to store updated information. The processor is adapted to receive the information from the non-volatile memory after the non-volatile memory and the processor are communicatively coupled. The instrument is adapted to remain operable to determine the concentration of the analyte after the non-volatile memory is communicatively decoupled from the processor. Preferably a programming strip includes the non-volatile memory and a communication bus. Preferably the opening is adapted to receive at least the programming strip and the processor is adapted to receive information from the non-volatile memory of the programming strip after the programming strip has been positioned at least partially in the opening such that the programming strip and processor are communicatively coupled via the communication bus.

Preferably the communication bus has exactly two lines. The first line is a power-data communication line and the second line is a ground line.

A method of communicating information to an instrument adapted to determine the analyte concentration of a fluid is disclosed. The method comprises the acts of: Providing the instrument having a processor and an opening adapted to couple the instrument to a non-volatile memory storing the information; Updating the processor with the information stored in the non-volatile memory; Removing the non-volatile memory from the instrument such that the non-volatile memory and the processor are communicatively decoupled; Operating the instrument, after the step of removing the non-volatile memory, to determine the analyte concentration.
Preferably a programming strip is used, which comprises the non-volatile memory, the non-volatile memory communicatively coupled to the processor when the programming strip is positioned at least partially in the opening. The processor is updated with the information stored in the non-volatile memory.

Preferably the non-volatile memory and the processor are communicatively coupled via a communication bus including exactly two lines. The first line is a power-data communication line and the second line is a ground line.

### BRIEF DESCRIPTION OF THE DRAWINGS

FIG. 1 a is a top view of a programming strip according to one embodiment of the present invention.

FIG. 1b is a side view of FIG. 1a.

FIG. 2 is a perspective view of a sensor-dispensing instrument in the open position showing a sensor pack being inserted according to one embodiment.

FIG. 3a is a front view of a sensor-dispensing instrument according to one embodiment.

FIG. 3b is a front view of a disposable cartridge with a programming strip and a plurality of reagent-test sensors according to one embodiment.

FIG. 3c is a top view of a reagent-test sensor according to one embodiment.

FIG. 4 is a front view of a sensor-dispensing instrument according to another embodiment.

FIG. 5a is a top view of a programming strip being inserted into an opening of the sensor-dispensing instrument of FIG. 4.

FIG. 5b is a side view of a programming strip being inserted into an opening of the sensor-dispensing instrument of FIG. 4.

While the invention is susceptible to various modifications and alternative forms, specific embodiments are shown by way of example in the drawings and are described in detail herein. It should be understood, however, that the invention is not intended to be limited to the particular forms disclosed. Rather, the invention is to cover all modifications, equivalents, and alternatives falling within the spirit and scope of the invention as defined by the appended claims.

### DETAILED DESCRIPTION OF THE ILLUSTRATED EMBODIMENTS

The present invention is directed to a programming strip that is adapted to provide updated information to a processor (e.g., a microprocessor) of an instrument and methods of using the same. The programming strip may include updated information related to reprogramming the existing algorithms, altering constants, or installing new algorithms in the instrument. Some non-limiting examples of information that may be sent to the processor include: (a) amending or replacing at least one of the existing algorithms that determines the concentration of the analyte; (b) adding or amending software code to address bugs in the software; (c) altering constants in the existing algorithms; and (d) altering limits in the programming such as amending the minimum amount of fluid needed to determine the analyte concentration. The instruments may be of various types including sensor-dispensing instruments. The instruments may be portable or table-top instruments.

The instruments are typically used to determine concentrations of analytes. Analytes that may be measured by the instrument include glucose, lipid profiles (e.g., cholesterol, triglycerides, LDL and HDL), microalbumin, hemoglobin A₁C, fructose, lactate, or bilirubin. The instruments are not limited, however, to determining these specific analytes and it is contemplated that other analyte concentrations may be determined. The analytes may be in, for example, a whole blood sample, a blood serum sample, a blood plasma sample, or other body fluids like ISF (interstitial fluid) and urine.

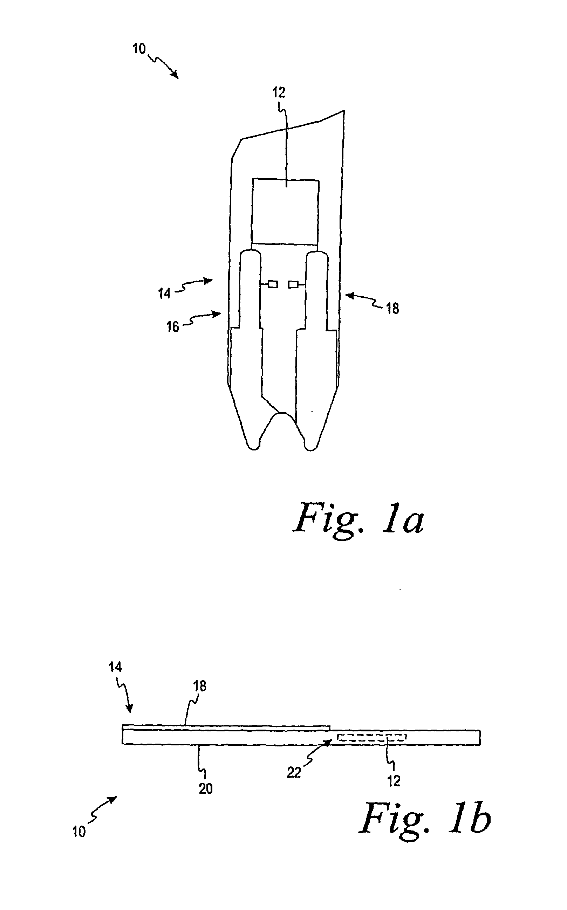

Referring to FIGs. 1a, b, a programming strip 10 is shown according to one embodiment. The programming strip 10 comprises a non-volatile memory 12 and a communication bus 14. In the embodiment illustrated, the communication bus 14 of FIG. 1 includes a power-data communication line 16 and a ground line 18. The non-volatile memory 12 stores the updated information and is communicatively coupled with a processor instrument via the communication bus 14.

A non-limiting example of non-volatile memory is an EEPROM. EEPROM (electronically erasable programmable read only memory) is a re-writable memory that does not require power to retain the contents of its memory. One example of memory that may be used in the programming strip is a chip marketed under the tradename DS2431, which is a 1024-bit, 1-wire® EEPROM chip made by Maxim Integrated Products, Inc./Dallas Semiconductor Corporation. Maxim Integrated Products is based in Sunnyvale, California, and Dallas Semiconductor Corporation is based in Dallas, Texas. The 1-wire® EEPROM chip is desirable because it uses only two wires. It is contemplated that other 1-wire® EEPROM chips may be used as the non-volatile memory.

It is contemplated that other EEPROMs may be used in the programming strip. It is contemplated that other non-volatile memory may be used in the programming strip, such as flash memory.

As shown in FIG. 1, the communication bus 14 is adapted to communicatively couple the memory 12 and the processor of the instrument. More specifically, according to one embodiment, the communication bus 14 is adapted to couple the memory 12 to the processor via a plurality of electrical connection pads of the instrument. The power-data communication line 16 enables two-way communication between the memory 12 and the processor of the instrument.

The communication bus 14 may be formed from a variety of materials. According to one embodiment, the communication bus comprises a plurality of traces. The plurality of traces may be an applied coating or painting such as carbon film. Alternative, the plurality of traces may be an applied coating or painting made of metal such as copper, tin, silver, gold, or combinations thereof. It is contemplated that the copper, tin, silver and gold may be in the form of alloys with other material. According to another embodiment, the communication bus may be a plurality of wires.

The communication bus 14 may have exactly two lines such as shown in FIG. 1a. It is contemplated that additional lines may be used to communicatively couple the memory and the processor of the instrument. For example, it is contemplated that exactly five lines may be used to communicatively couple the memory and the processor of the instrument.

The programming strip 10 typically is made of a polymeric material in which the memory and the conductive elements are placed thereon or therein. For example, the programming strip may be configured such as shown in FIG. 1b. As shown in FIG. 1b, the communication bus 14 is shown as being formed on top of the polymeric blank 20 and the non-volatile memory 12 is shown as being placed or embedded into an indentation 22. To further protect the non-volatile memory 12, it is desirable for the non-volatile memory to be placed into and mounted within the indentation 22. The non-volatile memory may be mounted in the indentation using, for example, an adhesive (e.g., an epoxy) or a soldering process. It is contemplated, however, that the non-volatile memory may reside on a top surface of the polymeric blank. In this embodiment, the non-volatile memory may be attached using, for example, an adhesive (e.g., an epoxy) or a soldering process. It is contemplated that the communication bus and memory may be formed or placed on the blank by other techniques.

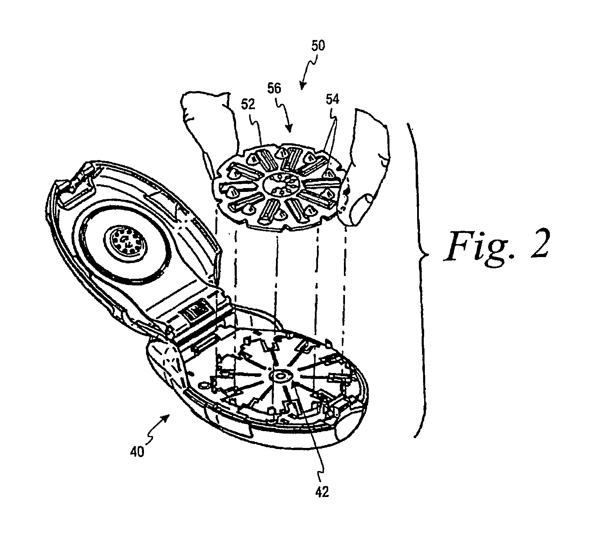

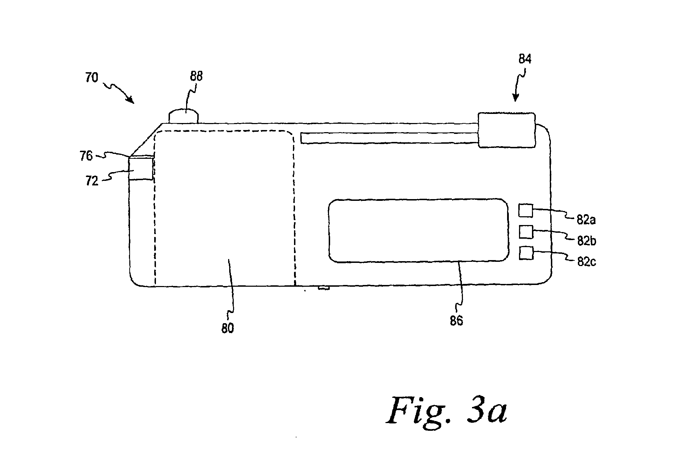

The memory 12 of the programming strip 10 is adapted to store the updated information and is adapted to be communicatively coupled with the instrument. According to one embodiment, the instrument is a sensor-dispensing instrument. Examples of sensor-dispensing instruments 40, 70 are shown in FIGs. 2,3a. It is contemplated that other sensor-dispensing instruments may be employed other than those depicted in FIGs. 2,3a.

More specifically, the memory 12 of the programming strip 10 is adapted to store the updated information and is adapted to be communicatively coupled with a processor (e.g., a microprocessor) of the instrument. The processor performs the computing, which includes interpreting and executing the instructions. The processor also may be a central processing unit (CPU).

Referring to FIGs. 2, 3a, each of the sensor-dispensing instruments 40, 70 includes a respective processor 42, 72. One example of a processor that may be used is an uPD78F0338 microprocessor by NEC Corporation of Japan. It is contemplated that other processors may be used such as selected processors made by companies such as Texas Instruments, Inc., Intel Corporation and Siemens AG. It is also contemplated that other processors may be used in the instrument.

The processors 42, 72 are adapted to identify the programming strip 10 as an internal memory-update device. More specifically, the processors 42, 72 read the memory 12, which results in identifying the type of updated information to be installed and the proper upload or reprogramming sequence may be initiated. After the update has been completed, the processor of the instrument will have the latest information.

According to one embodiment, the programming strip may be individually stored. The programming strip may be stored in a container such as a bottle or vial. The programming strip may also be packaged in a bottle or vial with a plurality of test sensors. According to one embodiment, the bottle contains one programming strip and from about 5 to about 100 test sensors. It is contemplated that the programming strip may be individually stored in other containers such as a packet.

According to another embodiment, the programming strip may be located in a disposable cartridge such as a sensor pack (e.g., a blister-type pack) by replacing one of the test sensors that is adapted to determine the analyte concentration. According to one embodiment, the test sensors are reagent-test sensors.

One example of a sensor pack or, more specifically, a blister-type pack is shown in FIG. 2. The blister-type pack 50 is shown in FIG. 2 being placed in the sensor-dispensing instrument 40. The blister-type pack 50 includes a programming strip 52 and a plurality of test sensors 54 that are individually stored in a respective one of the sensor cavities 56. It is contemplated that other sensor packs that individually hold the programming strip and plurality of test sensors may be used. The sensor pack of FIG. 2 (without the programming strip) is further described at U.S. Publication No. 2003/0032190 that published on February 13, 2003 and is entitled "Mechanical Mechanism for a Blood Glucose Sensor-Dispensing Instrument." It is contemplated that other sensor packs may be used.

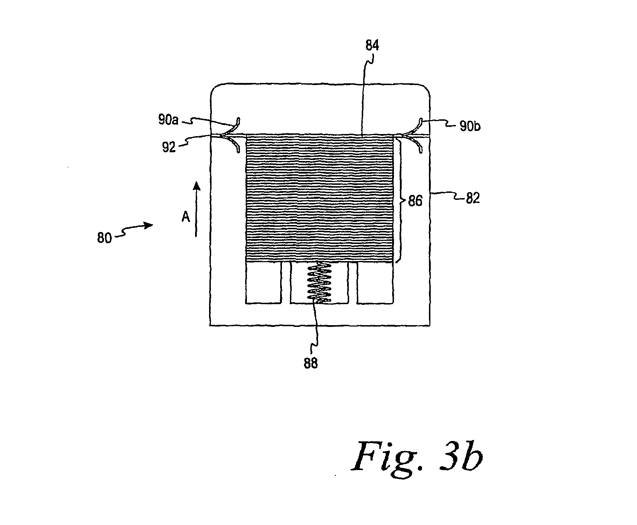

According to a further embodiment, the programming strip may also be located in a stack with a plurality of test sensors within a disposable cartridge such as shown in FIG. 3b. Referring to FIG. 3b, a disposable cartridge 80 includes a housing 82, a programming strip 84, and a plurality of reagent-test sensors 86. The programming strip 84 and the plurality of reagent-test sensors 86 is stacked in the cartridge 80.

The programming strip 84 and the plurality of stacked reagent-test sensors 86 are moved in the direction of arrow A via a spring 88. The cartridge 80 also includes a plurality of seals 90a,b that protects the stacked reagent-test sensors 90 from the humidity. The programming strip 84 and the plurality of reagent-test sensors 86, one at a time, exit the cartridge 80, via an opening 92. To promptly provide the new information to the processor of the instrument, the programming strip 84 is desirably located to be removed first from the cartridge 80.

The disposable cartridge 80 of FIG. 3b may be stored in the sensor-dispensing instrument 70 of FIG. 3a. It is contemplated that other cartridges may be used to contain the programming strip and the plurality of test sensors.

Typically, the sensor pack 50 and cartridge 80 of FIGs. 2,3b contain only one programming strip because all the updated information is stored in the memory of the programming strip. The cartridges typically contain from about 10 to about 50 test sensors and, more specifically, contain from about 25 to about 40 test sensors.

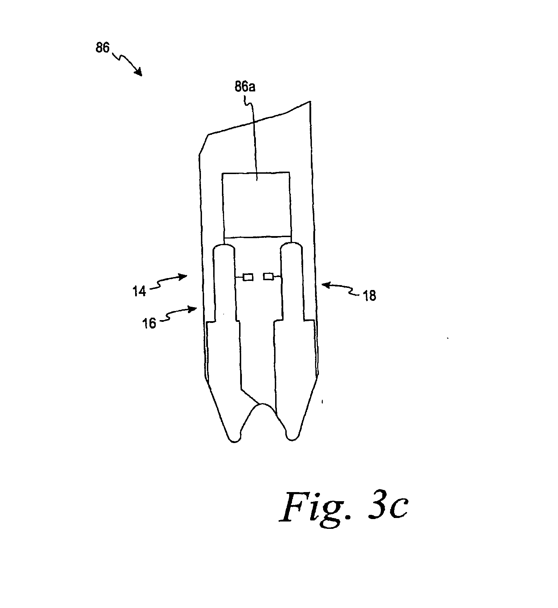

To reduce complexity, it may be desirable for the dimensions of the programming strip to be similar, if not identical, to the plurality of the test sensors that is adapted to determine the analyte concentration. For example, in one illustrated embodiment, the dimensions of the programming strip 10 of FIG. 1 are the same as the reagent-test sensors 86 shown in FIG. 3c. Specifically, the only difference between the programming strip 10 of FIG. 1 and the reagent-test sensors 86 of FIG. 3c is the replacement of the reagent-receiving area 86a with the memory 12 to form the programming strip 10. The reagent-receiving area and the memory may also have the same dimensions. According to another embodiment, the programming strip and the reagent-test sensors may be dimensioned differently. Similarly, the dimensions of the reagent-receiving area and the memory be different.

The methods of the present invention are desirable since the updated information may be provided to a processor of the instrument by a user. The user may be, for example, a user at home who needs to determine an analyte concentration (e.g., glucose) via the instrument.

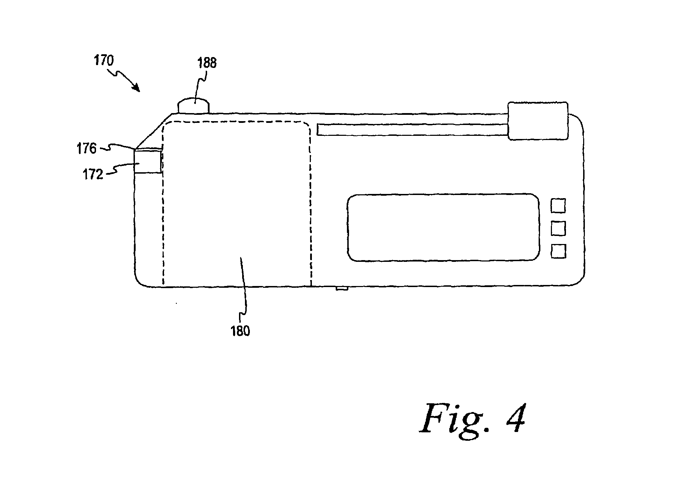

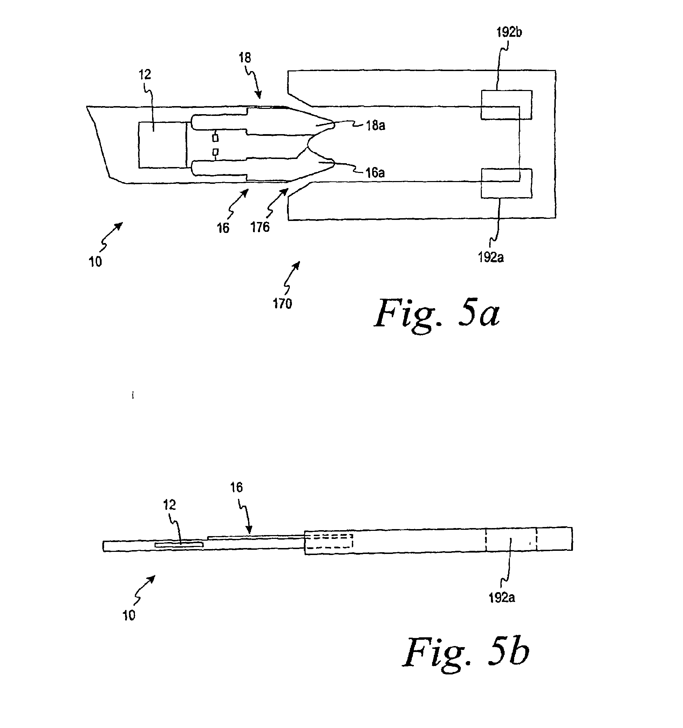

To provide the updated information to the processor of the instrument, the programming strip needs to be properly positioned in the instrument such that the programming strip is in communicatively coupled with the processor. According to one method, a user grasps the programming strip and properly positions it in an opening of the instrument. For example, referring to FIGs. 4 and 5a,b, a user may take a programming strip (e.g., programming strip 10) and position it in an opening 176 of sensor-dispensing instrument 170. The memory 12 of the programming strip 10 would then be communicatively coupled with the processor 172 of the sensor-dispensing instrument 170. In the illustrated embodiment, end portions 16a, 18a would contact a plurality of electrical contact pads 192a,b of the instrument 170 when the programming strip 10 is properly positioned in the opening 176. In the embodiment of FIG. 4, a cartridge 180 would not likely include a programming strip. Rather, the cartridge 180 includes only a plurality of test sensors.

To reduce costs, it is desirable to use an opening adapted to receive both the programming strip and the plurality of test sensors for determining the analyte concentration such as shown in FIG. 4 with opening 176. Additionally, by using the opening for both the programming strip and the test sensors, the instrument may be more compact by avoiding the need to have an opening for only the programming strip. It is contemplated, however, that separate openings adapted to receive a respective programming strip and test sensors may be formed in the instrument.

According to another method, the programming strip may automatically be moved and properly positioned without the user handling the programming strip. For example, referring to FIGs. 3a,3b, after a user positions the cartridge 80 in the sensor-dispensing instrument, the sensor-dispensing instrument automatically advances the programming strip 84 into an opening 76. The automatic advancing of the programming strip 84 may be initiated by several methods. For example, the automatic advancing may be initiated by pressing one of the plurality of buttons 82a-c, or pushing a pusher mechanism 84. The programming strip may be advanced by using a motor. It also may be initiated by the placing of the cartridge within the sensor-dispensing instrument.

The process of providing information from the memory of the programming strip to the processor of the instrument is typically performed in a short time period. An example of a short time period is generally from about 0.1 millisecond ("ms") to about 1 second and, more typically, from about 1 ms to about 50 ms. It is contemplated that the time needed to forward the updated information from the memory of the programming strip to the processor of the instrument may take longer, but this is, of course, less desirable.

According to one embodiment, the sensor-dispensing instrument 40, 70 may notify the user that an update or reprogramming sequence has occurred and the programming strip (e.g., programming strip 10) may be removed or discarded. For example, a display 86 of the sensor-dispensing instrument 70 in FIG. 3a may notify the user that an update sequence has occurred. One example of a display is a liquid-crystal display.

It is contemplated that the programming strip may be removed automatically by the instrument via an eject mechanism. In such a method, the test sensor is released forcefully. According to another method, a user manually releases the programming strip via a release mechanism 88 (FIG. 3a) or a release mechanism 188 (FIG. 4). In such embodiments, after the release mechanism is activated, the test sensors may be removed by tipping the instrument 70, 170 such that the programming strip falls from the instrument 70, 170 via gravity. Alternatively, after the release mechanism is activated, the test sensor may be removed by pulling it from the instrument. It is contemplated that the test sensor may be pulled from the instrument without using a release mechanism. It is contemplated that the programming strip may be removed by other techniques. After completing the reprogramming, the sensor-dispensing instrument will function as intended with, for example, the updated program, constants or algorithms.

### Embodiment A

A non-volatile memory that is adapted to provide updated information to an instrument. The instrument has a processor being adapted to determine the concentration of an analyte. The non-volatile memory is adapted to store the updated information and is adapted to be coupled communicatively to the processor of the instrument. The non-volatile memory is removable to communicatively decouple the non-volatile memory from the processor. The instrument remains operable to determine the concentration of the analyte after the non-volatile memory is removed. The communication bus has at least two lines that are adapted to assist in communicatively coupling the memory and the processor of the instrument.

### Embodiment B

The non-volatile memory of embodiment A wherein the non-volatile memory is communicatively coupled to the processor via a communication bus, the communication bus including exactly two lines.

### Embodiment C

The non-volatile memory of embodiment B wherein the communication bus comprises exactly five lines.

### Embodiment D

The non-volatile memory of embodiment A wherein the non-volatile memory is an EEPROM.

### Embodiment E

The non-volatile memory of embodiment A wherein the non-volatile memory is a flash memory.

### Embodiment F

The non-volatile memory of embodiment A wherein the non-volatile memory is included in a programming strip.

### Embodiment G

The non-volatile memory of embodiment F wherein the programming strip forms an indentation that receives the non-volatile memory.

### Embodiment H

The programming strip of embodiment A wherein the non-volatile memory is communicatively coupled to the processor via a communication bus, the communication bus comprises a plurality of traces.

### Embodiment I

A cartridge that is adapted to be used with a sensor-dispensing instrument, the instrument being adapted to determine the concentration of an analyte and includes the programming strip of embodiment F or G and a plurality of test sensors to be used by the instrument for determining the concentration of the analyte.

### Embodiment J

The cartridge of embodiment I wherein the plurality of test sensors is reagent-test sensors.

### Embodiment K

The cartridge of embodiment I wherein the cartridge is a sensor pack.

### Embodiment L

The cartridge of embodiment K wherein the sensor pack is a blister-type pack.

### Embodiment M

The cartridge of embodiment I wherein the plurality of test sensors is stacked.

### Embodiment N

The cartridge of embodiment M further including at least one seal to assist in protecting the plurality of test sensors.

### Embodiment O

The cartridge of embodiment I wherein the programming strip and the plurality of test sensors have the same dimensions.

### Embodiment P

The cartridge of embodiment I wherein the programming strip includes a communication bus having exactly two lines that are adapted to assist in communicatively coupling the non-volatile memory and the processor of the instrument, the first line being a power-data communication line and the second line being a ground line.

### Embodiment Q

An instrument for determining the analyte concentration of a fluid, the instrument comprising:
a non-volatile memory adapted to store updated information;
an opening adapted to couple the non-volatile memory to the instrument; and
a processor adapted to receive information from the non-volatile memory of the programming strip after the non-volatile memory and the processor are communicatively coupled. The instrument is adapted to remain operable to determine the concentration of the analyte after the non-volatile memory is communicatively decoupled from the processor.

### Embodiment R

An instrument according to embodiment Q wherein the non-volatile memory is included in a programming strip. The processor is adapted to receive the information from the non-volatile memory after the programming strip has been positioned at least partially in the opening such that the non-volatile memory and the processor are communicatively coupled.

### Embodiment S

The instrument of embodiment Q or R further including a plurality of test sensors.

### Embodiment T

The instrument of embodiment S wherein the opening is adapted to receive both the programming strip and the plurality of test sensors.

### Embodiment U

The instrument of embodiment Q wherein the fluid is blood and the analyte is glucose.

### Embodiment V

The instrument of embodiment R further including a plurality of electrical connection pads that is adapted to assist in communicatively coupling the programming strip and the processor.

### Embodiment W

An instrument according to embodiment Q, wherein the non-volatile memory is communicatively coupled to the processor via a communication bus including exactly two lines, the first line being a power-data communication line and the second line being a ground line.

### Embodiment W

The instrument of embodiment Q wherein the non-volatile memory is an EEPROM.

### Embodiment X

The instrument of embodiment Q wherein the non-volatile memory is a flash memory.

### Embodiment Y

A method of communicating information to an instrument adapted to determine the analyte concentration of a fluid, the method comprising the acts of:
providing the instrument having a processor and an opening adapted to couple the instrument and the processor to a non-volatile memory storing the information; and
updating the processor with the information stored in the non-volatile memory;
removing the non-volatile memory from the instrument such that the non-volatile memory and the processor are communicatively decoupled; and
operating the instrument, after the step of removing the non-volatile memory, to determine the analyte concentration.

### Embodiment Z

The method of embodiment Y including positioning a programming strip at least partially in the opening, the programming strip comprising the non-volatile memory, the non-volatile memory being communicatively coupled to the processor when the programming strip is at least partially in the opening.

### Embodiment AA

The method of embodiment Z wherein the positioning of the programming strip includes grasping the programming strip and placing the programming strip at least partially in the opening.

### Embodiment BB

The method of embodiment Z wherein the opening is adapted to receive the programming strip and a plurality of test sensors.

### Embodiment CC

The method of embodiment Z wherein the positioning of the programming strip is performed automatically by the instrument without the user handling the programming strip.

### Embodiment DD

The method of embodiment Y wherein the non-volatile memory and the processor are communicatively coupled via communication bus including exactly two lines, the first line being a power-data communication line and the second line being a ground line.

### Embodiment DD

The method of embodiment Y wherein the non-volatile memory is an EEPROM.

### Embodiment EE

The method of embodiment Y wherein the non-volatile memory is a flash memory.

## Claims

1. An instrument (40, 70, 170) for determining the analyte concentration of a fluid, the instrument (40, 70, 170) comprising:
an opening (76, 176) adapted to couple a programming strip (10, 52, 84) including a non-volatile memory (12) to the instrument (40, 70, 170), the non-volatile memory (12) being adapted to store information; and
a processor (42, 72) adapted to receive the information from the non-volatile memory (12) after the non-volatile memory (12) and the processor (42, 72) are communicatively coupled,
**characterized in**
the instrument (40, 70, 170) being adapted to remain operable to determine the concentration of the analyte after the programming strip (10, 52, 84) is removed from the instrument (40, 70, 179) and thereby the non-volatile memory (12) is communicatively decoupled from the processor (42, 72), and
the opening (76, 176) being further adapted to at least partially receive a test sensor (54, 86) for determining the concentration of an analyte.

2. The instrument (40, 70, 170) of claim 1 wherein the opening (76, 176) is adapted to at least partially receive the programming strip (10, 52, 84), which has the same dimensions as the test sensor (54, 86).

3. The instrument (40, 70, 170) of claim 1 wherein the fluid is blood and the analyte is glucose.

4. The instrument (40, 70, 170) of one of the claims 13 to 15 further including a plurality of electrical connection pads (192a, 192b) that is adapted to assist in communicatively coupling the non-volatile memory (12) included in the programming strip (10, 52, 84) and the processor (42, 72).

5. The instrument (40, 70, 170) of one of the claims 1 to 4 wherein the non-volatile memory (12) is communicatively coupled to the processor (42, 72) via a communication bus (14) including exactly two lines, the first line being a power-data communication line (16) and the second line being a ground line (18).

6. The instrument (40, 70, 170) of one of the claims 1 to 5 wherein the non-volatile memory (12) is a flash memory or an EPROM.

7. A method of communicating information to an instrument (40, 70, 170) adapted to determine the analyte concentration of a fluid, the method comprising the acts of:
providing the instrument (40, 70, 170) having a processor (42, 72) and an opening (76, 176) adapted to couple the instrument (40, 70, 170) and the processor (42, 72) to a non-volatile memory (12) storing the information; and
updating the processor (42, 72) with the information stored in the non-volatile memory (12);
**characterized in** the further acts of
positioning a programming strip (10, 52, 84) at least partially in the opening (76, 176), the programming strip (10, 52, 84) comprising the non-volatile memory (12), the non-volatile memory (12) being communicatively coupled to the processor (42, 72) when the programming strip (10, 52, 84) is at least partially in the opening (76, 176), the opening (76, 176) being adapted to receive both the programming strip (10, 52, 84) and a test sensor (54, 86),
removing the non-volatile memory (12) from the instrument (40, 70, 70) such that the non-volatile memory (12) and the processor (42, 72) are communicatively decoupled; and
operating the instrument (40, 70, 170), after the step of removing the non-volatile memory (12), to determines the analyte concentration.

8. The method of claim 7 wherein the programming strip (10, 52, 84) has the same dimensions as the test sensor (54, 86).

9. The method of claim 7 wherein the positioning of the programming strip (10, 52, 84) is performed automatically by the instrument (40, 70, 170) without the user handling the programming strip (10, 52, 84).

10. The method of one of the claims 7 to 9, wherein the non-volatile memory (12) and the processor (42, 72) are communicatively coupled via a communication bus (14) including exactly two lines, the first line being a power-data communication line (16) and the second line being a ground line (18).

11. The method of one of the claims 7 to 9 wherein the non-volatile memory (12) is a flash memory or an EPROM.

12. A programming strip (10, 52, 84) including a non-volatile memory (12) that is adapted to provide information to an instrument (40, 70, 170) according to one of the claims 1 to 6,
**characterized in**
the programming strip (10, 52. 84) being adapted to be received at least partially by the same opening (76, 176) in the instrument (40, 70. 170) as a test sensor (54, 86), which is adapted for determining the concentration of an analyte.

13. The programming strip (10, 52, 84) of claim 12 including a communication bus (14) having exactly two lines or exactly five lines for communicatively coupling the non-volatile memory (12) and the processor (42, 72) of the instrument (40, 70, 170).

14. The programming strip (10, 52, 84) of claim 12 including a communication bus (14) having exactly two lines, the first line being a power-data communication line (16) and the second line being a ground line (18).

15. The programming strip (10, 52, 84) of claim 12 wherein the non-volatile memory (12) is received by an indentation (22) formed by the programming strip (10, 52, 84).

16. The programming strip (10, 52, 84) of one of the claims 1 to 4 wherein the non-volatile memory (12) is a flash memory or an EPROM.

17. The programming strip (10, 52, 84) of claim 12 including a communication bus (14) having a plurality of traces for communicatively coupling the non-volatile memory (12) and the processor (42, 72) of the instrument (40, 70, 174).

18. A cartridge (50, 80) that is adapted to be used with a sensor-dispensing instrument (40, 70, 170), the instrument (40, 70, 170) being adapted to determine the concentration of an analyte, **characterized in that** the cartridge comprises:
a programming strip (10, 52, 84) according to one of the claims l2 to 18; and
a plurality of test sensors (54, 86) to be used by the instrument (40, 70, 170) for determining the concentration of the analyte.

19. The cartridge (50, 80) of claim 18 wherein the cartridge (50, 80) is a sensor pack.

20. The cartridge (50, 80) of claim 19 wherein the sensor pack is a blister-type pack (50).

21. The cartridge (50, 80) of claim 18 or 19 wherein the plurality of test sensors (54, 86) is stacked.

22. The cartridge (50, 80) of one of the claims 18 to 21 further including at least one seal (90a, b) to assist in protecting the plurality of test sensors (54, 86).

23. The cartridge (50, 80) of one of the claims l8 to 22 wherein the programming strip (10, 52, 84) and the plurality of test sensors (54, 86) have the same dimensions.

## Patentansprüche

1. Instrument (40, 70, 170) zum Bestimmen der Analytkonzentration eines Fluids, wobei das Instrument (40, 70, 170) das Folgende aufweist:
eine Öffnung (76, 176), welche dafür geeignet ist, einen Programmierstreifen (10, 52, 84), der einen nichtflüchtigen Speicher (12) aufweist, mit dem Instrument (40, 70, 170) zu verbinden, wobei der nichtflüchtige Speicher (12) dafür geeignet ist, Informationen zu speichern; und
einen Prozessor (42, 72), welcher dafür geeignet ist, die Informationen aus dem nichtflüchtigen Speicher (12) zu empfangen, nachdem der nichtflüchtige Speicher (12) und der Prozessor (42, 72) kommunikativ verbunden worden sind,
**dadurch gekennzeichnet, dass**
das Instrument (40, 70, 170) dafür geeignet ist, weiter die Konzentration des Analyten zu bestimmen, nachdem der Programmierstreifen (10, 52, 84) aus dem Instrument (40, 70, 170) entfernt worden ist und **dadurch** der nichtflüchtige Speicher (12) kommunikativ von dem Prozessor (42, 72) entkoppelt worden ist, und
die Öffnung (76, 176) ferner dafür geeignet ist, zumindest teilweise einen Testsensor (54, 86) zum Bestimmen der Konzentration eines Analyten aufzunehmen.

2. Instrument (40, 70, 170) nach Anspruch 1, wobei die Öffnung (76, 176) dafür geeignet ist, zumindest teilweise den Programmierstreifen (10, 52, 84) aufzunehmen, welcher dieselben Abmessungen wie der Testsensor (54, 86) aufweist.

3. Instrument (40, 70, 170) nach Anspruch 1, wobei es sich bei dem Fluid um Blut und bei dem Analyten um Glucose handelt.

4. Instrument (40, 70, 170) nach einem der Ansprüche 13 bis 15, welches ferner mehrere elektrische Kontaktflecken (192a, 192b) aufweist, die dafür geeignet sind, dabei zu helfen, den nichtflüchtigen Speicher (12), der in dem Programmierstreifen (10, 52, 84) enthalten ist, und den Prozessor (42, 72) kommunikativ zu verbinden.

5. Instrument (40, 70, 170) nach einem der Ansprüche 1 bis 4, wobei der nichtflüchtige Speicher (12) über einen Kommunikationsbus (14), der genau zwei Leitungen aufweist, kommunikativ mit dem Prozessor (42, 72) verbunden ist, wobei es sich bei der ersten Leitung um eine Strom-/Datenkommunikationsleitung (16) und bei der zweiten Leitung um eine Erdungsleitung (18) handelt.

6. Instrument (40, 70, 170) nach einem der Ansprüche 1 bis 5, wobei es sich bei dem nichtflüchtigen Speicher (12) um einen Flash-Speicher oder einen EPROM handelt.

7. Verfahren zum Übermitteln von Informationen an ein Instrument (40, 70, 170), welches dafür geeignet ist, die Analytkonzentration in einem Fluid zu bestimmen, wobei das Verfahren die folgenden Schritte aufweist:
Bereitstellen des Instruments (40, 70, 170), welches einen Prozessor (42, 72) und eine Öffnung (76, 176) aufweist, die dafür geeignet ist, das Instrument (40, 70, 170) und den Prozessor (42, 72) mit einem nichtflüchtigen Speicher (12) zu verbinden, in welchem die Informationen gespeichert sind; und
Aktualisieren des Prozessors (42, 72) mit den Informationen, die in dem nichtflüchtigen Speicher (12) gespeichert sind;
**gekennzeichnet durch** die folgenden weiteren Schritte:
Anordnen eines Programmierstreifens (10, 52, 84) zumindest teilweise in der Öffnung (76, 176), wobei der Programmierstreifen (10, 52, 84) den nichtflüchtigen Speicher (12) aufweist, wobei der nichtflüchtige Speicher (12) kommunikativ mit dem Prozessor (42, 72) verbunden ist, wenn sich der Programmierstreifen (10, 52, 84) zumindest teilweise in der Öffnung (76, 176) befindet, wobei die Öffnung (76, 176) dafür geeignet ist, sowohl den Programmierstreifen (10, 52, 84) als auch einen Testsensor (54, 86) aufzunehmen,
Entfernen des nichtflüchtigen Speichers (12) aus dem Instrument (40, 70, 170), so dass der nichtflüchtige Speicher (12) und der Prozessor (42, 72) kommunikativ entkoppelt werden; und
Bestimmen der Analytkonzentration **durch** das Instrument (40, 70, 170) nach dem Schritt des Entfernens des nichtflüchtigen Speichers (12).

8. Verfahren nach Anspruch 7, wobei der Programmierstreifen (10, 52, 84) dieselben Abmessungen wie der Testsensor (54, 86) aufweist.

9. Verfahren nach Anspruch 7, wobei die Positionierung des Programmierstreifens (10, 52, 84) automatisch durch das Instrument (40, 70, 170) erfolgt, ohne dass der Benutzer den Programmierstreifen (10, 52, 84) befördert.

10. Verfahren nach einem der Ansprüche 7 bis 9, wobei der nichtflüchtige Speicher (12) und der Prozessor (42, 72) über einen Kommunikationsbus (14), der genau zwei Leitungen aufweist, kommunikativ verbunden sind, wobei es sich bei der ersten Leitung um eine Strom-/Datenkommunikationsleitung (16) und bei der zweiten Leitung um eine Erdungsleitung (18) handelt.

11. Verfahren nach einem der Ansprüche 7 bis 9, wobei es sich bei dem nichtflüchtigen Speicher (12) um einen Flash-Speicher oder einen EPROM handelt.

12. Programmierstreifen (10, 52, 84), welcher einen nichtflüchtigen Speicher (12) aufweist, der dafür geeignet ist, einem Instrument (40, 70, 170) nach einem der Ansprüche 1 bis 6 Informationen bereitzustellen,
**dadurch gekennzeichnet, dass**
der Programmierstreifen (10, 52, 84) dafür geeignet ist, zumindest teilweise von derselben Öffnung (76, 176) in dem Instrument (40, 70, 170) als ein Testsensor (54, 86), der dafür geeignet ist, die Konzentration eines Analyten zu bestimmen, aufgenommen zu werden.

13. Programmierstreifen (10, 52, 84) nach Anspruch 12, welcher einen Kommunikationsbus (14), der genau zwei Leitungen oder genau fünf Leitungen aufweist, zum kommunikativen Verbinden des nichtflüchtigen Speichers (12) mit dem Prozessor (42, 72) des Instruments (40, 70, 170) aufweist.

14. Programmierstreifen (10, 52, 84) nach Anspruch 12, welcher einen Kommunikationsbus (14) aufweist, der genau zwei Leitungen aufweist, wobei es sich bei der ersten Leitung um eine Strom-/Datenkommunikationsleitung (16) und bei der zweiten Leitung um eine Erdungsleitung (18) handelt.

15. Programmierstreifen (10, 52, 84) nach Anspruch 12, wobei der nichtflüchtige Speicher (12) von einer Einkerbung (22) aufgenommen wird, die durch den Programmierstreifen (10, 52, 84) gebildet wird.

16. Programmierstreifen (10, 52, 84) nach einem der Ansprüche 1 bis 4, wobei es sich bei dem nichtflüchtigen Speicher (12) um einen Flash-Speicher oder einen EPROM handelt.

17. Programmierstreifen (10, 52, 84) nach Anspruch 12, welcher einen Kommunikationsbus (14) aufweist, der mehrere Leiterbahnen zum kommunikativen Verbinden des nichtflüchtigen Speichers (12) mit dem Prozessor (42, 72) des Instruments (40, 70, 170) aufweist.

18. Kassette (50, 80), welche dafür geeignet ist, mit einem Sensor-Ausgabe-Instrument (40, 70, 170) benutzt zu werden, wobei das Instrument (40, 70, 170) dafür geeignet ist, die Konzentration eines Analyten zu bestimmen, **dadurch gekennzeichnet, dass** die Kassette das Folgende aufweist:
einen Programmierstreifen (10, 52, 84) nach einem der Ansprüche 12 bis 18 und
mehrere Testsensoren (54, 86), die von dem Instrument (40, 70, 170) verwendet werden können, um die Konzentration des Analyten zu bestimmen.

19. Kassette (50, 80) nach Anspruch 18, wobei es sich bei der Kassette (50, 80) um eine Sensorpackung handelt.

20. Kassette (50, 80) nach Anspruch 19, wobei es sich bei der Sensorpackung um eine Packung (50) des Blister-Typs handelt.

21. Kassette (50, 80) nach Anspruch 18 oder 19, wobei die mehreren Testsensoren (54, 86) gestapelt sind.

22. Kassette (50, 80) nach einem der Ansprüche 18 bis 21, welche ferner mindestens eine Dichtung (90a, b) aufweist, um dabei zu helfen, die mehreren Testsensoren (54, 86) zu schützen.

23. Kassette (50, 80) nach einem der Ansprüche 18 bis 22, wobei der Programmierstreifen (10, 52, 84) und die mehreren Testsensoren (54, 86) dieselben Abmessungen aufweisen.

## Revendications

1. Instrument (40, 70, 170) pour déterminer la concentration en analytes d'un fluide, l'instrument (40, 70, 170) comprenant :
une ouverture (76, 176) conçue pour assurer le couplage à l'instrument (40, 70, 170) d'une bande de programmation (10, 52, 84) incluant une mémoire rémanente (12), la mémoire rémanente (12) étant conçue pour stocker des informations ; et
un processeur (42, 72) conçu pour recevoir les informations à partir de la mémoire rémanente (12), une fois que la mémoire rémanente (12) et le processeur (42, 72) sont couplés de façon communicante, **caractérisé en ce que**
l'instrument (40, 70, 170) est conçu de façon à rester opérationnel pour déterminer la concentration de l'analyte une fois que la bande de programmation (10, 52, 84) est enlevée de l'instrument (40, 70, 170) et par conséquent la mémoire rémanente (12) est découplée de façon communicante du processeur (42, 72), et
l'ouverture (76, 176) est conçue en outre de façon à recevoir au moins partiellement un capteur de test (54, 86) pour déterminer la concentration d'un analyte.

2. Instrument (40, 70, 170) selon la revendication 1, l'ouverture (76, 176) étant conçue pour recevoir au moins partiellement la bande de programmation (10, 52, 84), qui a les mêmes dimensions que le capteur de test (54, 86).

3. Instrument (40, 70, 170) selon la revendication 1, le fluide étant du sang et l'analyte étant du glucose.

4. Instrument (40, 70, 170) selon l'une des revendications 13 à 15, comportant en outre une pluralité de plots de connexion électrique (192a, 192b) qui est conçue de façon à contribuer au couplage communicant entre la mémoire rémanente (12) intégrée à la bande de programmation (10, 52, 84) et le processeur (42, 72).

5. Instrument (40, 70, 170) selon l'une des revendications 1 à 4, la mémoire rémanente (12) étant couplée de façon communicante au processeur (42, 72) par l'intermédiaire d'un bus de communication (14) incluant exactement deux lignes, la première ligne étant une ligne de communication énergie-données (16) et la deuxième ligne étant une ligne de terre (18).

6. Instrument (40, 70, 170) selon l'une des revendications 1 à 5, la mémoire rémanente (12) étant une mémoire Flash ou une EPROM.

7. Procédé de communication d'informations à un instrument (40, 70, 170) conçu pour déterminer la concentration en analytes d'un fluide, le procédé comprenant les opérations consistant à :
mettre à disposition l'instrument (40, 70, 170) doté d'un processeur (42, 72) et d'une ouverture (76, 176) conçue de façon à coupler l'instrument (40, 70, 170) et le processeur (42, 72) à une mémoire rémanente (12) laquelle stocke les informations ; et
actualiser le processeur (42, 72) avec les informations stockées dans la mémoire rémanente (12) ;
**caractérisé en ce que** les opérations supplémentaires consistent à :
positionner une bande de programmation (10, 52, 84) au moins partiellement dans l'ouverture (76, 176), la bande de programmation (10, 52, 84) comprenant la mémoire rémanente (12), la mémoire rémanente (12) étant couplée de façon communicante au processeur (42, 72) lorsque la bande de programmation (10, 52, 84) se trouve au moins partiellement dans l'ouverture (76, 176), l'ouverture (76, 176) étant conçue pour recevoir à la fois la bande de programmation (10, 52, 84) et un capteur de test (54, 86),
enlever la mémoire rémanente (12) de l'instrument (40, 70, 170) de sorte que la mémoire rémanente (12) et le processeur (42, 72) sont découplés en termes de communication ; et
faire fonctionner l'instrument (40, 70, 170) après l'étape d'enlèvement de la mémoire rémanente (12) afin de déterminer la concentration de l'analyte.

8. Procédé selon la revendication 7, la bande de programmation (10, 52, 84) ayant les mêmes dimensions que le capteur de test (54, 86).

9. Procédé selon la revendication 7, le positionnement de la bande de programmation (10, 52, 84) étant effectué automatiquement par l'instrument (40, 70, 170) sans que l'utilisateur manipule la bande de programmation (10, 52, 84).

10. Procédé selon l'une des revendications 7 à 9, la mémoire rémanente (12) et le processeur (42, 72) étant couplés de façon communicante par l'intermédiaire d'un bus de communication (14) incluant exactement deux lignes, la première ligne étant une ligne de communication énergie-données (16) et la deuxième ligne étant une ligne de terre (18).

11. Procédé selon l'une des revendications 7 à 9, la mémoire rémanente (12) étant une mémoire Flash ou une EPROM.

12. Bande de programmation (10, 52, 84) englobant une mémoire rémanente (12) qui est conçue de façon à fournir des informations à un instrument (40, 70, 170) selon l'une des revendications 1 à 6,
**caractérisée en ce que**
la bande de programmation (10, 52, 84) est conçue de façon à être reçue au moins partiellement par la même ouverture (76, 176) ménagée dans l'instrument (40, 70, 170) qu'un capteur de test (54, 86) lequel est conçu de façon à déterminer la concentration d'un analyte.

13. Bande de programmation (10, 52, 84) selon la revendication 12, englobant un bus de communication (14) possédant exactement deux lignes, ou exactement cinq lignes, pour coupler de façon communicante la mémoire rémanente (12) et le processeur (42, 72) de l'instrument (40, 70, 170).

14. Bande de programmation (10, 52, 84) selon la revendication 12, englobant un bus de communication (14) possédant exactement deux lignes, la première ligne étant une ligne de communication énergie-données (16) et la deuxième ligne étant une ligne de terre (18).

15. Bande de programmation (10, 52, 84) selon la revendication 12, la mémoire rémanente (12) étant reçue par une encoche (22) formée par la bande de programmation (10, 52, 84).

16. Bande de programmation (10, 52, 84) selon l'une des revendications 1 à 4, la mémoire rémanente (12) étant une mémoire Flash ou une EPROM.

17. Bande de programmation (10, 52, 84) selon la revendication 12, englobant un bus de communication (14) possédant une pluralité de traces pour assurer le couplage communicant entre la mémoire rémanente (12) et le processeur (42, 72) de l'instrument (40, 70, 170).

18. Cartouche (50, 80) conçue en vue d'une utilisation avec un instrument de distribution à capteurs (40, 70, 170), l'instrument (40, 70, 170) étant conçu de façon à déterminer la concentration d'un analyte, **caractérisée en ce que** la cartouche comprend :
une bande de programmation (10, 52, 84) selon l'une des revendications 12 à 18 ; et
une pluralité de capteurs de test (54, 86) destinés à être utilisés par l'instrument (40, 70, 170) pour déterminer la concentration de l'analyte.

19. Cartouche (50, 80) selon la revendication 18, la cartouche (50, 80) étant un emballage à capteurs.

20. Cartouche (50, 80) selon la revendication 19, l'emballage à capteurs étant un emballage de type bulle (50).

21. Cartouche (50, 80) selon la revendication 18 ou 19, la pluralité de capteurs de test (54, 86) se présentant en empilement.

22. Cartouche (50, 80) selon l'une des revendications 18 à 21, comportant en outre au moins un scellement (90a, b) pour contribuer à protéger la pluralité de capteurs de test (54, 86).

23. Cartouche (50, 80) selon l'une des revendications 18 à 22, la bande de programmation (10, 52, 84) et la pluralité de capteurs de test (54, 86) ayant les mêmes dimensions.
